(19) Europäisches Patentamt
European Patent Office
Office européen des brevets

(11) Veröffentlichungsnummer: **0 405 267 A1**

(12) **EUROPÄISCHE PATENTANMELDUNG**

(21) Anmeldenummer: **90111391.0**

(22) Anmeldetag: **16.06.90**

(51) Int. Cl.[5]: **C07D 249/08**, C07D 233/60, C07D 405/06, C07D 405/14, C07D 409/06, C07D 409/14, C07D 407/06, C07D 303/08, C07D 307/12, C07D 333/16, C07C 49/163

(30) Priorität: **30.06.89 DE 3921481**

(43) Veröffentlichungstag der Anmeldung: **02.01.91 Patentblatt 91/01**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Scherkenbeck, Jürgen, Dr.**
**Auf dem Bruch 49**
**D-5090 Leverkusen 3(DE)**
Erfinder: **Frie, Monika, Dr.**
**Im alten Driesch 1**
**D-5068 Odenthal-Osenau(DE)**
Erfinder: **Stroech, Klaus, Dr.**
**Rolsberger Strasse 22**
**D-5650 Solingen 19(DE)**
Erfinder: **Himmler, Thomas, Dr.**
**Bonhöfferstrasse 20**
**D-5000 Köln 80(DE)**
Erfinder: **Ludwig, Georg-W., Dr.**
**Bethelstrasse 22**
**D-4150 Krefeld 1(DE)**
Erfinder: **Brandes, Wilhelm, Dr.**
**Eichendorffstrasse 3**
**D-5653 Leichlingen 1(DE)**
Erfinder: **Dutzmann, Stefan, Dr.**
**Leinenweberweg 33**
**D-4000 Düsseldorf 13(DE)**

(54) **Hydroxyethyl-cyclopropyl-azolyl-Derivate.**

(57) Neue Hydroxyethyl-cyclopropyl-azolyl-Derivate der Formel

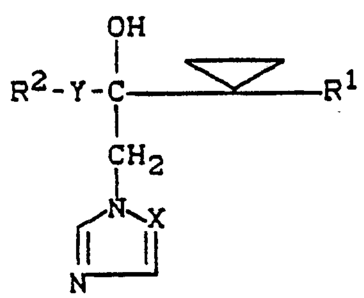

(I)

in welcher
$R^1$, $R^2$, X und Y die in der Beschreibung angegebene Bedeutung haben,
sowie deren Säureadditions-Salze und Metallsalz-Komplexe,
mehrere Verfahren zur Herstellung der neuen Stoffe und der Verwendung als Mikrobizide im Pflanzenschutz und im Materialschutz.

Neue Zwischenprodukte, Verfahren zu deren Herstellung und deren Verwendung zur Synthese von Stoffen

EP 0 405 267 A1

der Formel (I).

# HYDROXYETHYL-CYCLOPROPYL-AZOLYL-DERIVATE

Die vorliegende Erfindung betrifft neue Hydroxyethyl-cyclopropyl-azolyl-Derivate, mehrere Verfahren zur deren Herstellung und deren Verwendung als Mikrobizide im Pflanzenschutz und im Materialschutz.

Es ist bereits bekannt, daß zahlreiche Hydroxyalkyl-azolyl-Derivate fungizide Eigenschaften besitzen (vgl. EP-OS 0 298 332). So lassen sich zum Beispiel 1-(4-Trifluormethoxyphenyl)-3-(1-chlor-cycloprop-1-yl)-4-(1,2,4-triazol -1-yl)-but-1-en-3-ol und 1-(4-Chlorphenyl)-3-(1-chlor-cycloprop-1-yl)-4-(1,2,4-triazol-1-yl)-butan-3-ol zur Bekämpfung phytopathogener Pilze verwenden. Die Wirksamkeit dieser Stoffe ist gut; bei niedrigen Aufwandmengen läßt die Wirksamkeit allerdings in manchen Fällen zu wünschen übrig.

Es wurden nun neue Hydroxyethyl-cyclopropyl-azolyl-Derivate der Formel

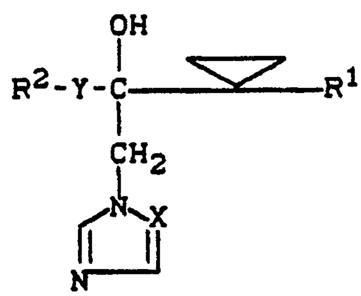

(I)

in welcher

$R^1$ für Halogen, Phenyl oder die Gruppierung -Z-$R^3$ steht, worin

Z für Sauerstoff, Schwefel, SO oder $SO_2$ steht und

$R^3$ für Alkyl, Phenyl oder Benzyl steht,

$R^2$ für Alkenyl, gegebenenfalls substituiertes Furyl, gegebenenfalls substituiertes Thienyl oder den Rest der Formel

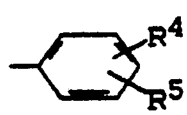

steht, worin

$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und

$R^5$ für Wasserstoff oder Halogen steht, oder

$R^4$ und $R^5$ in ortho-Position verknüpft sind und gemeinsam für -O-$CH_2$-O- stehen,

oder

$R^2$ für den Rest der Formel

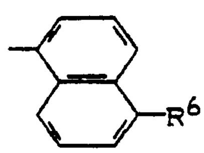

steht,

worin

$R^6$ für Difluormethoxy, 1,1,2,2-Tetrafluorethoxy oder 1,1,2 -Trifluor-2-chlor-ethoxy steht,

oder

$R^2$ auch für gegebenenfalls durch Halogen und/oder Phenyl substituiertes Phenyl steht, wenn Y für die Gruppierung

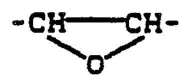

steht,
X für Stickstoff oder eine CH-Gruppe steht und
Y für die Gruppierungen

-CH=CH-, -C≡C- oder $-CH_2-CH_2-$,

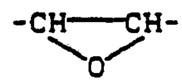

steht,
sowie deren Säureadditionssalze und Metallsalzkomplexe gefunden.

Die erfindungsgemäßen Stoffe enthalten ein asymmetrisch substituiertes Kohlenstoffatom. Sie können daher in optischen Isomerenformen anfallen. Darüber hinaus können diejenigen Stoffe der Formel (I), in denen Y für eine -CH = CH-Gruppe oder für eine

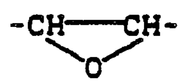 Gruppe

steht, auch in Form von cis- bzw. trans-Isomeren vorliegen. Die Erfindung betrifft sowohl die einzelnen Isomeren als auch deren Gemische.

Weiterhin wurde gefunden, daß man Hydroxyethyl-cyclopropyl-azolyl-Derivate der Formel (I) sowie deren Säureadditions-Salze und Metallsalz-Komplexe erhält, wenn man

a) Oxirane der Formel

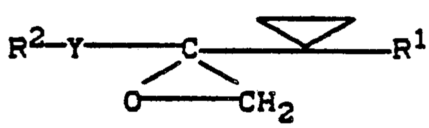 (II)

in welcher
$R^1$, $R^2$ und Y die oben angegebene Bedeutung haben,
mit Azolen der Formel

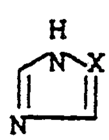 (III)

in welcher
X die oben angegebene Bedeutung hat,
in Gegenwart eines Säurebindemittels und in Gegenwart eines Verdünnungsmittels umsetzt,
oder

b) Ethanol-Derivate der Formel

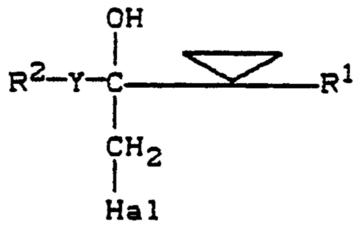

(IV)

in welcher
$R^1$, $R^2$ und Y die oben angegebene Bedeutung haben, und
Hal für Chlor, Brom oder Iod steht,
mit Azolen der Formel

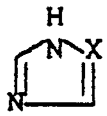

(III)

in welcher
X die oben angegebene Bedeutung hat,
in Gegenwart eines Säurebindemittels und in Gegenwart ein Verdünnungsmittels umsetzt,
oder
c) Hydroxyethyl-cyclopropyl-azolyl-Derivate der Formel

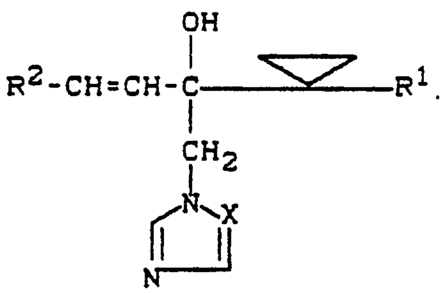

(Ia)

in welcher
$R^1$, $R^2$ und X die oben angegebene Bedeutung haben,
mit einem zur Expoxidierung geeigneten Reagenz gegebenenfalls in Gegenwart eines Verdünnungsmittels umsetzt, und gegebenenfalls anschließend an die so erhaltenen Verbindungen der Formel (I) eine Säure oder ein Metallsalz addiert.

Schließlich wurde gefunden, daß die neuen Hydroxyethyl-cyclopropyl-azolyl-Derivate der Formel (I) sowie deren Säureadditions-Salze und Metallsalz-Komplexe sehr gute mikrobizide Eigenschaften besitzen und sowohl im Pflanzenschutz als auch im Materialschutz eingesetzt werden können.

Überraschenderweise zeigen die erfindungsgemäßen Stoffe bei der Bekämpfung von phytopathogenen Pilzen eine deutlich bessere Wirksamkeit als 1-(4-Trifluormethoxyphenyl)-3-(1-chlor-cycloprop-1-yl)-4-(1,2,4-triazol-1-yl)-but-1-en-ol und 1-(4-chlorphenyl)-3-(1-chlorcycloprop-1-yl)-4-(1,2,4-triazol-1-yl)-butan-3-ol, welches konstitutionell ähnliche, vorbekannte Verbindungen gleicher Wirkungsrichtung sind.

Die erfindungsgemäßen Hydroxyethyl-cyclopropyl-azolyl-Derivate sind durch die Formel (I) allgemein definiert. In dieser Formel stehen vorzugsweise
$R^1$ für Fluor, Chlor, Brom, Phenyl oder für die Gruppierung $-Z-R^3$, worin
Z für Sauerstoff, Schwefel, SO oder $SO_2$ steht und
$R^3$ für Alkyl mit 1 bis 6 Kohlenstoffatomen, Phenyl oder Benzyl steht
$R^2$ für geradkettiges oder verzweigtes Alkenyl mit 2 bis 8 Kohlenstoffatomen, gegebenenfalls durch Halogen oder Furyl substituiertes Furyl, für gegebenenfalls durch Halogen substituiertes Thienyl oder für den Rest der Formel

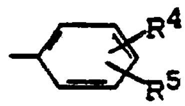

,

worin

$R^4$ für Difluormethoxy, 1,1,2,2-Tetrafluorethoxy, 1,1,2-Trifluor-2-chlor-ethoxy, Cyano, Formyl, Alkoximinoalkyl mit 1 bis 4 Kohlenstoffatomen in der Alkoxygruppe und 1 bis 4 Kohlenstoffatomen in der Alkylgruppe, Carbalkoxy mit 1 bis 4 Kohlenstoffatomen in der Alkoxygruppe, Dialkoxyalkyl mit 1 bis 4 Kohlenstoffatomen in jeder Alkoxygruppe und 1 bis 4 Kohlenstoffatomen in der Alkylgruppe, gegebenenfalls im Phenylteil durch Fluor und/oder Chlor substituiertes Phenoxyalkyl mit 1 bis 4 Kohlenstoffatomen in der Alkylgruppe oder für gegebenenfalls im Phenylteil durch Fluor und/oder Chlor substituiertes Benzyloxy steht und

$R^5$ für Wasserstoff, Fluor, Chlor oder Brom steht, oder

$R^4$ und $R^5$ in ortho-Position verknüpft sind und gemeinsam für $-O-CH_2-O-$ stehen,

oder

$R^2$ für den Rest der Formel

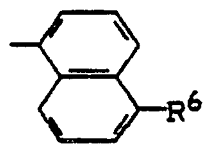

,

worin

$R^6$ für Difluormethoxy, 1,1,2,2-Tetrafluorethoxy oder 1,1,2 -Trifluor-2-chlor-ethoxy steht,

oder

$R^2$ für gegebenenfalls durch Fluor, Chlor und/oder Phenyl substituiertes Phenyl, wenn Y für die Gruppierung

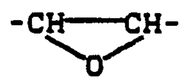

steht,

X für Stickstoff oder eine CH-Gruppe und

Y für die Gruppierungen

$-CH=CH-$, $-C\equiv C-$ oder $-CH_2-CH_2-$,

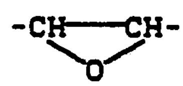

.

Besonders bevorzugt sind diejenigen Verbindungen der Formel (I), in denen

$R^1$ für Fluor, Chlor, Brom, Phenyl oder für die Gruppierung $-Z-R^3$, worin

Z für Sauerstoff, Schwefel, SO oder $SO_2$ steht und

$R^3$ für Alkyl mit 1 bis 4 Kohlenstoffatomen, Phenyl oder Benzyl steht

$R^2$ für geradkettiges oder verzweigtes Alkenyl mit 3 bis 6 Kohlenstoffatomen, gegebenenfalls durch Fluor, Chlor, Brom oder Furyl substituiertes Furyl, für gegebenenfalls durch Fluor, Chlor oder Brom substituiertes Thienyl, oder für den Rest der Formel

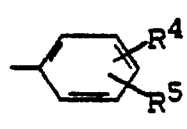

steht,

worin

$R^4$ für Difluormethoxy, 1,1,2,2-Tetrafluorethoxy, 1,1,2-Trifluor-2-chlor-ethoxy, Cyano, Formyl, Alkoximinoalkyl

mit 1 oder 2 Kohlenstoffatomen in der Alkoxygruppe und 1 oder 2 Kohlenstoffatomen in der Alkylgruppe, Carbalkoxy mit 1 oder 2 Kohlenstoffatomen in der Alkoxygruppe, Dialkoxyalkyl mit 1 oder 2 Kohlenstoffatomen in jeder Alkoxygruppe und 1 oder 2 Kohlenstoffatomen in der Alkylgruppe, gegebenenfalls im Phenylteil durch Fluor und/oder Chlor substituiertes Phenoxyalkyl mit 1 oder 2 Kohlenstoffatomen in der Alkylgruppe oder für gegebenenfalls im Phenylteil durch Fluor und/oder Chlor substituiertes Benzyloxy steht und

$R^5$ für Wasserstoff, Fluor, Chlor oder Brom steht, oder

$R^4$ und $R^5$ in ortho-Position verknüpft sind und gemeinsam für $-O-CH_2-O-$ stehen,

oder

$R^2$ für den Rest der Formel

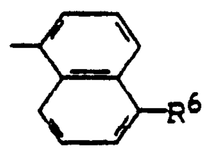

steht,

worin

$R^6$ für Difluormethoxy, 1,1,2,2-Tetrafluorethoxy oder 1,1,2 -Trifluor-2-chlor-ethoxy steht,

oder

$R^2$ auch für gegebenenfalls durch Fluor, Chlor und/oder Phenyl substituiertes Phenyl steht, wenn Y für die Gruppierung

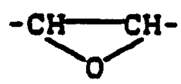

steht,

X für Stickstoff oder eine CH-Gruppe steht und

Y für die Gruppierungen

$-CH=CH-$, $-C\equiv C-$ oder $-CH_2-CH_2-$,

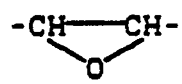

steht.

Bevorzugte erfindungsgemäße Verbindungen sind auch Additionsprodukte aus Säuren und denjenigen Hydroxyethyl-cyclopropyl-azolyl-Derivaten der Formel (I), in denen $R^1$, $R^2$, X und Y die Bedeutungen haben, die bereits vorzugsweise für diese Reste genannt wurden.

Zu den Säuren, die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren,wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Hydroxyethyl-cyclopropyl-azolyl-Derivaten der Formel (I), in denen $R^1$, $R^2$, X und Y die Bedeutungen haben, die bereits vorzugsweise für diese Reste genannt wurden.

Hierbei sind Salze des Kupfers, Zinks, Mangans, Magnesium, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren,

wie z.B. die Chlorwasserstoffsäure und die Bromwasserstoffsäure, ferner Phosphorsäure, Salpetersäure und Schwefelsäure.

Verwendet man 2-(1-Chlor-cycloprop-1-yl)-2-[4-(2-chlor-1,1,2-trifluor-ethoxy)-phenyl-ethenyl]-oxiran und 1,2,4-Triazol als Ausgangsstoffe, so kann der Verlauf des erfindungsgemäßen Verfahrens (a) durch das folgende Formelschema veranschaulicht werden:

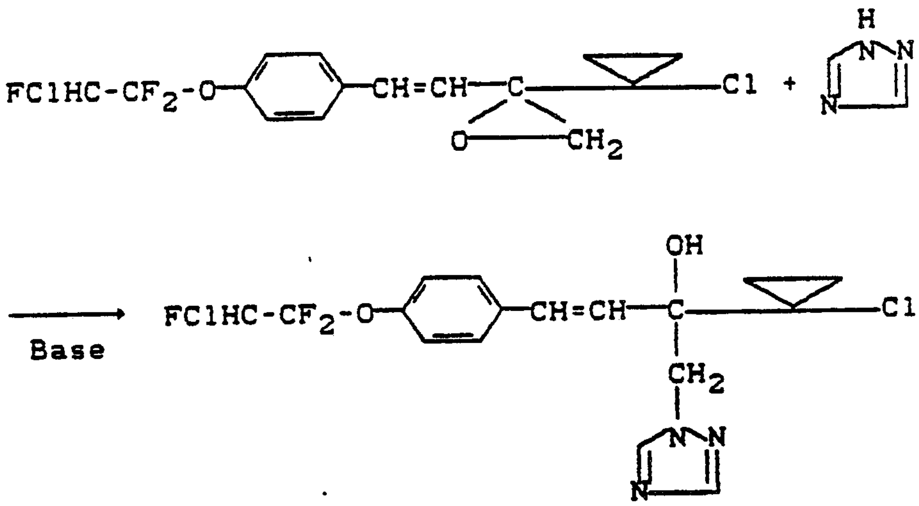


Verwendet man 1-Chlor-2-(1-chlor-cycloprop-1-yl)-4-(thiophen-2-yl)-but-3-in-2-ol und 1,2,4-Triazol als Ausgangsstoffe, so kann der Verlauf des erfindungsgemäßen Verfahrens (b) durch das folgende Formelschema veranschaulicht werden:

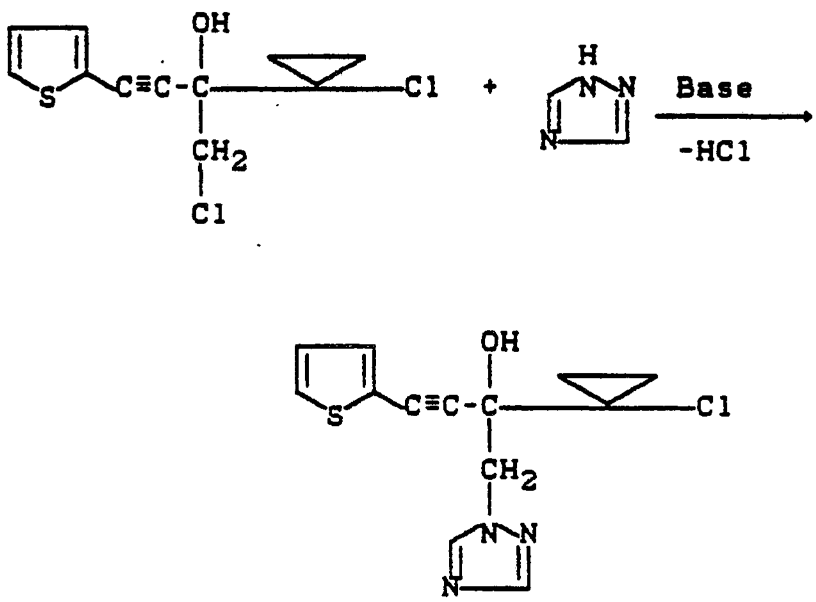


Verwendet man 4-Biphenylyl-2-(1-chlor-cycloprop-1-yl)-1-(1,2,4-triazol-1-yl)-but-3-en-2-ol als Ausgangsstoff und 3-Chlor-perbenzoesäure als Epoxidierungsreagenz, so kann der Verlauf des erfindungsgemäßen Verfahrens (c) durch das folgende Formelschema veranschaulicht werden:

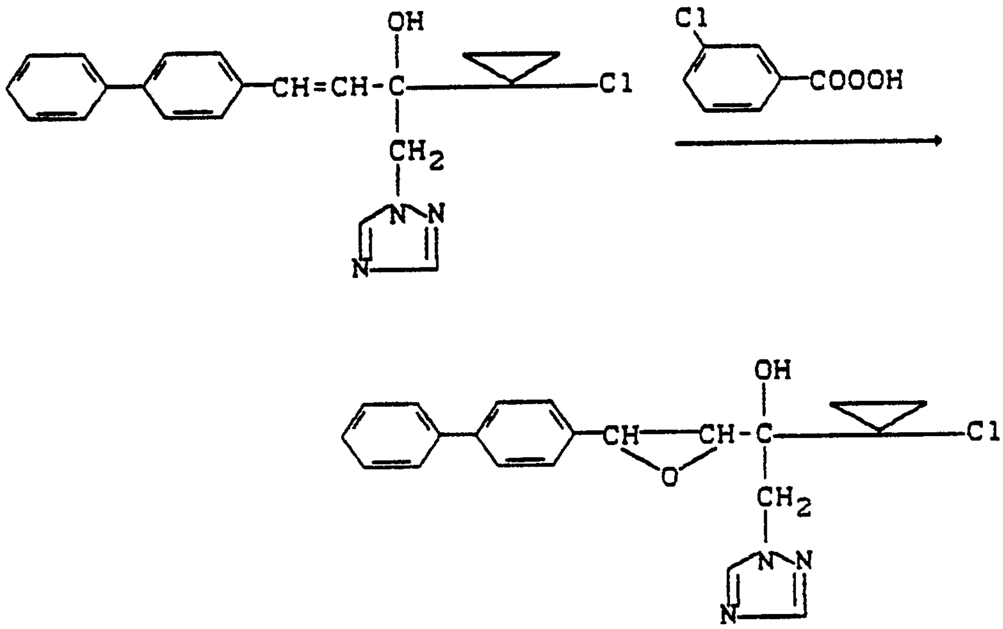

Die bei dem erfindungsgemäßen Verfahren (a) als Ausgangsstoffe benötigten Oxirane sind durch die Formel (II) allgemein definiert. In dieser Formel haben $R^1$, $R^2$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Oxirane der Formel (II) sind bisher noch nicht bekannt. Sie lassen sich herstellen, indem man

d) Cyclopropylketone der Formel

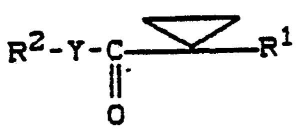 (V)

in welcher

$R^1$, $R^2$ und Y die oben angegebene Bedeutung haben,

entweder

α) mit Dimethyloxosulfonium-methylid der Formel

$$\overset{\delta\oplus\,\delta\ominus}{(CH_3)_2SOCH_2} \qquad (VI)$$

oder

β) mit Dimethylsulfonium-methylid der Formel

$$\overset{\delta\oplus\,\delta\ominus}{(CH_3)_2S\ CH_2} \qquad (VII)$$

in Gegenwart eines Verdünnungsmittels umsetzt, oder indem man

e) Carbinole der Formel

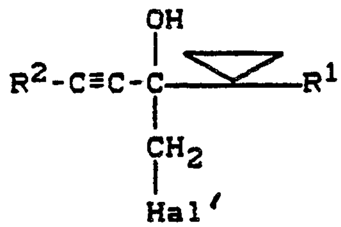

(IVa)

in welcher

$R^1$ und $R^2$ die oben angegebene Bedeutung haben und

Hal′ für Chlor oder Brom steht,

mit Basen in Gegenwart eines Verdünnungsmittels umsetzt.

Die bei der Durchführung des Verfahrens (d) als Ausgangsstoffe benötigten Cyclopropylketone der Formel (V) sind bisher noch nicht bekannt. Sie lassen sich herstellen, indem man

f) Aldehyde der Formel

$R^2$-CHO (VIII)

in welcher

$R^2$ die oben angegebene Bedeutung hat,

mit Methyl-cyclopropyl-ketonen der Formel

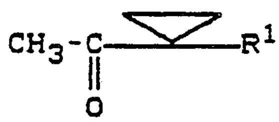

(IX)

in welcher

$R^1$ die oben angegebene Bedeutung hat,

in Gegenwart eines Katalysators und in Gegenwart eines Verdünnungsmittels umsetzt und gegebenenfalls die dabei entstehenden Cyclopropylketone der Formel

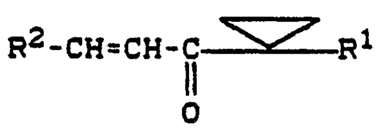

(V-a)

in welcher

$R^1$ und $R^2$ die oben angegebene Bedeutungen haben

entweder

α) in Gegenwart eines Katalysators und in Gegenwart eines Verdünnungsmittels hydriert,

oder

β) mit einem zur Epoxidierung geeigneten Reagenz gegebenenfalls in Gegenwart eines Verdünnungsmittels umsetzt,

oder indem man

g) Acetylene der Formel

$R^2$-C≡CH (X)

in welcher

$R^2$ die oben angegebene Bedeutung hat,

mit Säurehalogeniden der Formel

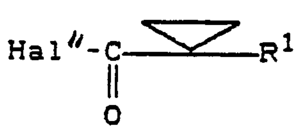

(XI)

in welcher
$R^1$ die oben angegebene Bedeutung hat und
Hal″ für Chlor oder Brom steht,
in Gegenwart eines Katalysators und in Gegenwart eines Verdünnungsmittels umsetzt.

Die bei dem Verfahren (f) als Ausgangsstoffe benötigten Aldehyde der Formel (VIII) sind allgemein bekannte Verbindungen der organischen Chemie.

Die außerdem bei der Durchführung des Verfahrens (f) als Reaktionskomponenten benötigten Methyl-cyclopropyl-ketone der Formel (IX) sind bekannt oder lassen sich nach prinzipiell bekannten Verfahren herstellen (vgl. Synthesis 1977, 189).

Als Katalysatoren kommen bei der Durchführung der ersten Stufe des Verfahrens (f) alle für derartige Kondensationen üblichen Reaktionsbeschleuniger in Frage. Vorzugsweise verwendbar sind basische Substanzen, z.B. Alkalimetallhydroxide, wie Natriumhydroxid oder Kaliumhydroxid.

Als Verdünnungsmittel kommen bei der Durchführung der ersten Stufe des Verfahrens (f) alle für derartige Umsetzungen üblichen inerten organischen Solventien in Betracht. Vorzugsweise verwendbar sind Alkohole, wie Methanol, Ethanol, Isopropanol, n-Butanol und tert.-Butanol.

Die Reaktionstemperaturen können bei der Durchführung der ersten Stufe des Verfahrens (f) innerhalb eines größeren Bereiches variiert werden. Im allgemeinen arbeitet man bei Temperaturen zwischen 0° C und 100° C, vorzugsweise zwischen 10° C und 80° C.

Die erste Stufe des Verfahrens (f) wird im allgemeinen unter Normaldruck durchgeführt. Es ist aber auch möglich, unter erhöhtem oder vermindertem Druck zu arbeiten.

Bei der Durchführung der ersten Stufe des Verfahrens (f) setzt man auf 1 Mol an Methyl-cyclopropylketon der Formel (IX) 1 Mol an Aldehyd der Formel (VIII) sowie eine katalytische Menge an Reaktionsbeschleuniger ein. Es ist jedoch auch möglich, die eine oder andere Komponente in einem Überschuß zu verwenden. Die Aufarbeitung erfolgt nach üblichen Methoden. Im allgemeinen geht man so vor, daß man die in festem Zustand anfallenden Reaktionsprodukte absaugt und nach gegebenenfalls vorheriger Reinigung für die weiteren Umsetzungen verwendet.

In der zweiten Stufe des Verfahrens (f) werden beim Arbeiten nach der Variante α die Cyclopropylketone der Formel (V-a) in Gegenwart eines Katalysators und eines Verdünnungsmittels mit Wasserstoff hydriert. Man arbeitet dabei in flüssiger Phase unter Verwendung eines suspendierten, pulverförmigen Hydrierkatalysators (heterogen) oder unter Verwendung eines im Verdünnungsmittel löslichen Katalysatorkomplexes (homogen). Die Durchführung der Hydrierung kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen bei der Durchführung der zweiten Stufe des Verfahrens (f, Variante α) inerte organische Lösungsmittel in Frage. Hierzu gehören vorzugsweise Alkohole, wie Methanol, Ethanol, Isopropanol oder Ethylenglykol; Ether, wie Diethylether, Diisopropylether, Ethylenglykolmonomethylether, Ethylenglykoldimethylether, Dioxan oder Tetrahydrofuran; gesättigte Kohlenwasserstoffe, wie n-Heptan oder Cyclohexan; aromatische Kohlenwasserstoffe, wie Toluol; sowie Ester, wie Essigsäureethylester.

Die Reaktionstemperaturen können bei der Durchführung der zweiten Stufe des Verfahrens (f, Variante α) in einem größeren Bereich variiert werden. Im allgemeinen arbeitet man bei Temperaturen zwischen 0° C und 150° C, vorzugsweise zwischen 20° C und 120° C.

Die Hydrierungen in der zweiten Stufe des Verfahrens (f, Variante α) werden bei Normaldruck oder auch unter erhöhtem Druck durchgeführt. Im allgemeinen arbeitet man unter Drucken zwischen 1 und 150 bar, vorzugsweise zwischen 5 und 60 bar.

Die Aufarbeitung erfolgt bei der Durchführung der zweiten Stufe des Verfahrens (f, Variante α) nach üblichen Methoden.

Als Epoxidierungsmittel kommen bei der Durchführung der zweiten Stufe des Verfahrens (f, Variante β) alle für derartige Umsetzungen geeigneten Epoxidierungsreagenzien in Betracht. Vorzugsweise verwendbar sind Persäuren, wie Perbenzoesäure, 3-chlor-perbenzoesäure und Peressigsäure, und außerdem auch Wasserstoffperoxid.

Als Verdünnungsmittel kommen bei der Durchführung der zweiten Stufe des Verfahrens (f, Variante β) alle für derartige Umsetzungen üblichen Solventien in Frage. Arbeitet man mit Persäuren, so kommen vorzugsweise inerte organische Lösungsmittel, wie Dichlormethan, in Betracht. Dient Wasserstoffperoxid als Epoxidierungsmittel, so werden vorzugsweise Wasser oder Gemische aus Wasser und inerten organischen Solventien, wie Dichlormethan, verwendet.

Die Reaktionstemperaturen können bei der Durchführung der zweiten Stufe des Verfahrens (f, Variante β) innerhalb eines bestimmten Bereiches variiert werden. Im allgemeinen arbeitet man bei Temperaturen

zwischen 0° C und 40° C, vorzugsweise zwischen 10° C und 30° C.

Bei der Durchführung der zweiten Stufe des Verfahrens (f, Variante β) geht man im allgemeinen so vor, daß man auf 1 Mol an cyclopropylketon der Formel (V-a) eine äquivalente Menge oder auch einen Überschuß an Epoxidierungsmittel einsetzt. Die Aufarbeitung erfolgt nach üblichen Methoden.

Die bei der Durchführung des Verfahrens (g) als Ausgangsstoffe benötigten Acetylene der Formel (X) sind bekannt oder lassen sich nach prinzipiell bekannten Verfahren in einfacher Weise herstellen.

Die bei der Durchführung des Verfahrens (g) als Reaktionskomponenten benötigten Säurehalogenide der Formel (XI) sind ebenfalls bekannt oder lassen sich nach prinzipiell bekannten Verfahren herstellen.

Als Katalysatoren kommen bei der Durchführung des Verfahrens (g) alle für derartige Umsetzungen üblichen Reaktionsbeschleuniger in Frage. Vorzugsweise verwendbar sind Kupfersalze, wie z.B. Kupferiodid.

Als Verdünnungsmittel kommen bei der Durchführung des Verfahrens (g) alle für derartige Umsetzungen üblichen inerten organischen Solventien in Betracht. Vorzugsweise verwendbar sind Ether, wie Tetrahydrofuran und Diethylether.

Die Reaktionstemperaturen können bei der Durchführung des Verfahrens (g) innerhalb eines bestimmten Bereiches variiert werden. Im allgemeinen arbeitet man bei Temperaturen zwischen -78° C und +50° C, vorzugsweise zwischen -78° C und +40° C.

Bei der Durchführung des Verfahrens (g) setzt man auf 1 Mol an Acetylen der Formel (X) im allgemeinen 1 bis 1,2 Mol an Säurehalogenid der Formel (XI) sowie Katalysator ein. Die Aufarbeitung erfolgt nach üblichen Methoden.

Das bei dem Verfahren (d) als Reaktionskomponente benötigte Dimethyl-oxo-sulfonium-methylid der Formel (VI) ist bekannt (vgl. J. Am. Chem. Soc. 87 , 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei dem Verfahren (d) außerdem als Reaktionskomponente in Betracht kommende Dimethylsulfonium-methylid der Formel (VII) ist ebenfalls bekannt (vgl. Heterocycles 8 , 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kalium-hydroxid, in Gegenwart eines Verdünnungsmittels, wie tert.-Butanol oder Dimethylsulfoxid erzeugt.

Als Verdünnungsmittel kommen bei der Durchführung des Verfahrens (d) inerte organische Solventien in Frage.

Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des Verfahrens (d) in einem größeren Bereich variiert werden. Im allgemeinen arbeitet man zwischen 0° C und 100° C, vorzugsweise zwischen 10° C und 60° C.

Bei der Durchführung des Verfahrens (d) setzt man auf 1 Mol an Cyclopropyl-keton der Formel (V) im allgemeinen 1 bis 3 Mol an Dimethyloxosulfonium-methylid der Formel (VI) bzw. an Dimethylsulfonium-methylid der Formel (VII) ein. Die Isolierung der Oxirane der Formel (II) erfolgt nach üblichen Methoden.

Die bei der Durchführung des Verfahrens (e) als Ausgangsstoffe benötigten Carbinole der Formel (IV-a) sind bisher noch nicht bekannt. Sie lassen sich herstellen, indem man

h) Halogenketone der Formel

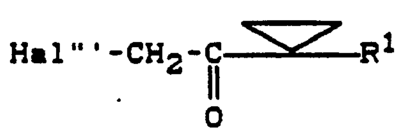

(XII)

in welcher

$R^1$ die oben angegebene Bedeutung hat und

Hal''' für Chlor oder Brom steht,

mit Acetylensalzen der Formel

$R^2-C\equiv\overset{\ominus}{C}\ Me^{\oplus}$ (XIII)

in welcher

$R^2$ die oben angegebene Bedeutung hat und
Me für ein Äquivalent eines Metallkations steht,
gegebenenfalls in Gegenwart eines Säurebindemittels und in Gegenwart eines Verdünnungsmittels umsetzt.

Die bei dem Verfahren (h) als Ausgangsstoffe benötigten Halogenketone der Formel (XII) sind bekannt (vgl. EP-OS 0 298 332).

Die bei dem Verfahren (h) als Reaktionskomponenten benötigten Acetylen-Salze sind durch die Formel (XIII) allgemein definiert. In dieser Formel hat $R^2$ vorzugsweise diejenigen Bedeutungen, die bereits im Zusammenhang mit der Beschreibung der erfindungsgemäßen Stoffe der Formel (I) vorzugsweise für diesen Rest genannt wurden. Me steht vorzugsweise für ein Lithiumkation oder für ein Äquivalent eines Cer(III)-Kations.

Die Acetylen-Salze der Formel (XIII) sind bekannt oder lassen sich nach prinzipiell bekannten Verfahren herstellen.

Als Säurebindemittel kommen bei der Durchführung des Verfahrens (h) alle üblichen Säureakzeptoren in Frage.

Als Verdünnungsmittel kommen bei der Durchführung des Verfahrens (h) alle üblichen inerten organischen Solventien in Betracht. Vorzugsweise verwendbar sind aromatische Kohlenwasserstoffe, wie Toluol, und außerdem Ether, wie Diethylether, Tetrahydrofuran, tert.-Butyl-methyl-ether und Gemische dieser Ether.

Die Reaktionstemperaturen können bei der Durchführung des Verfahrens (h) innerhalb eines größeren Bereiches variiert werden. Im allgemeinen arbeitet man bei Temperaturen zwischen -100 °C und +100 °C, vorzugsweise zwischen -80 °C und +50 °C.

Das Verfahren (h) wird im allgemeinen unter Normaldruck durchgeführt. Es ist aber auch möglich, unter erhöhtem oder vermindertem Druck zu arbeiten.

Bei der Durchführung des Verfahrens (h) setzt man auf 1 Mol an Halogenketon der Formel (XII) im allgemeinen 1 bis 3 Mol an Acetylensalz der Formel (XIII) ein. Die Aufarbeitung erfolgt nach üblichen Methoden.

Als Basen kommen bei der Durchführung des Verfahrens (e) alle üblicherweise für derartige Umsetzungen geeigneten organischen und anorganischen Säurebindemittel in Betracht. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung des Verfahrens (e) alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des Verfahrens (e) innerhalb eines bestimmten Bereiches variiert werden. Im allgemeinen arbeitet man bei Temperaturen zwischen -20 °C und +100 °C, vorzugsweise zwischen 0 °C und 60 °C.

Bei der Durchführung des Verfahrens (e) arbeitet man im allgemeinen unter Normaldruck. Es ist jedoch auch möglich, unter erhöhtem oder vermindertem Druck zu arbeiten.

Bei der Durchführung des Verfahrens (e) setzt man auf 1 Mol an Carbinol der Formel (VI-a) im allgemeinen 1 bis 3 Mol an Base ein. Die Aufarbeitung erfolgt nach üblichen Methoden.

Die bei der Durchführung des erfindungsgemäßen Verfahrens (a) als Reaktionskomponenten benötigten Azole der Formel (III) sind allgemein bekannte Verbindungen der organischen Chemie.

Als Säurebindemittel kommen bei der Durchführung des erfindungsgemäßen Verfahrens (a) alle üblichen Säureakzeptoren in Frage. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, außerdem tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN), 1,8-Diaza-bicyclo[5.4.0]undec-7-en (DBU) und 1,4-Diaza-bicyclo[2.2.2]octan (DABCO).

Als Verdünnungsmittel kommen bei der Durchführung des erfindungsgemäßen Verfahrens (a) alle üblichen inerten organischen Solventien in Betracht. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsäuretriamid.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und

200° C, vorzugsweise zwischen 50 und 150° C.

Bei der Durchführung des erfindungsgemäßen Verfahrens (a) setzt man vorzugsweise auf 1 Mol an Oxiran der Formel (II) 1 bis 4 Mol an Azol der Formel (III) und 1 bis 2 Mol an Base ein. Die Isolierung der Endprodukte erfolgt in üblicher Weise.

Die bei der Durchführung des erfindungsgemäßen Verfahrens (b) als Ausgangsstoffe benötigten Ethanol-Derivate sind durch die Formel (IV) allgemein definiert. In dieser Formel haben $R^1$, $R^2$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Ethanol-Derivate der Formel (IV) sind bisher noch nicht bekannt. Sie lassen sich nach dem Verfahren (h) herstellen, sofern es sich um diejenigen Stoffe handelt, in denen Y für -C≡C- steht. Aus diesen Stoffen der Formel (IV-a) lassen sich die übrigen Ethanol-Derivate der Formel (IV) herstellen, indem man die Dreifachbindung nach üblichen Methoden ganz oder teilweise hydriert, oder indem man diejenigen Stoffe, in denen Y für -CH=CH- steht, nach üblichen Methoden epoxidiert (vgl. zweite Stufe des Verfahrens (f, Variante $\beta$)).

Bei der Durchführung des erfindungsgemäßen Verfahrens (b) kommen als Säurebindemittel alle üblichen Säureakzeptoren in Betracht. Vorzugsweise verwendbar sind alle diejenigen Säurebindemittel, die bereits im Zusammenhang mit der Beschreibung des erfindungsgemäßen Verfahrens (a) genannt wurden.

Als Verdünnungsmittel kommen bei der Durchführung des erfindungsgemäßen Verfahrens (b) alle üblichen inerten organischen Solventien in Frage. Vorzugsweise verwendbar sind alle diejenigen Verdünnungsmittel, die bereits im Zusammenhang mit der Beschreibung des erfindungsgemäßen Verfahrens (a) genannt wurden.

Die Reaktionstemperaturen können auch bei der Durchführung des erfindungsgemäßen Verfahrens (b) innerhalb eines größeren Bereiches variiert werden. Im allgemeinen arbeitet man bei Temperaturen zwischen 0° C und 120° C, vorzugsweise zwischen 20° C und 100° C.

Das erfindungsgemäße Verfahren (b) wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens (b) setzt man im allgemeinen auf 1 Mol an Ethanol-Derivat der Formel (IV) 1,5 bis 3 Mol an Azol der Formel (III) sowie 3 bis 6 Mol an Säurebindemittel ein. Die Aufarbeitung erfolgt nach üblichen Methoden.

Bei der Durchführung des erfindungsgemäßen Verfahrens (c) werden als Ausgangsstoffe Hydroxyethyl-cyclopropyl-azolyl-Derivate der Formel (I a) eingesetzt. Diese lassen sich nach den erfindungsgemäßen Verfahren (a) oder (b) herstellen. Die Epoxidierung nach dem erfindungsgemäßen Verfahren (c) wird in gleicher Weise durchgeführt wie die Epoxidierung in der zweiten Stufe des Verfahrens (f, Variante $\beta$).

Die nach den erfindungsgemäßen Verfahren erhältlichen Hydroxyethyl-cyclopropyl-azolyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Obergegriffe fallen, genannt:

Xanthomonas-Arten, wie Xanthomonas oryzae;

Pseudomonas-Arten, wie Pseudomonas lachrymans;

Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus ;
(Konidienform: Drechslera, Syn: Helminthosporium);
Uromyces-Arten, wie Uromyces appendiculatus;
Puccinia-Arten, wie Puccinia recondita;
Tilletio-Arten, wie Tilletia caries;
Ustilago avenae; wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich im Pflanzenschutz insbesondere zur Bekämpfung von Getreide-und Reiskrankheiten. So lassen sich Mehltau-und Rost-Krankheiten, wie Erysiphe, Leptosphaeria nodorum, Pyrenophora teres, Cochliobolus sativus und Fusarium nivale an Getreide sowie Pyricularia und Pellicularia an Reis besonders gut bekämpfen. Die Stoffe können außerdem gegen Botrytis cinerea, echten Mehltau an Gurken sowie gegen Venturia an Äpfeln verwendet werden; sie besitzen außerdem eine sehr gute in -vitro-Wirkung.

Im Materialschutz lassen sich die erfindungsgemäßen Wirkstoffe zum Schutz von technischen Materialien einsetzen. Unter technischen Materialien sind in diesem Zusammenhang nicht lebende Materialien zu verstehen, die für die Verwendung in der Technik zubereitet worden sind. Beispielsweise können technische Materialien, die durch die erfindungsgemäßen Wirkstoffe von mikrobieller Veränderung oder Zerstörung geschützt werden sollen, Klebestoffe, Leime, Papier, Karton, Textilien, Leder, Holz, Anstrichmittel, Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt, besonders bevorzugt Holz.

Als Mikroorganismen, die einen Abbau oder eine Veränderung der technischen Materialien bewirken können seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,

Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wid hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugssweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %,

am Wirkungsort erforderlich.

Die Herstellung und die Verwendung der erfindungsgemäßen Wirkstoffe geht aus den nachfolgenden Beispielen hervor.

Herstellungsbeispiele

Beispiel 1

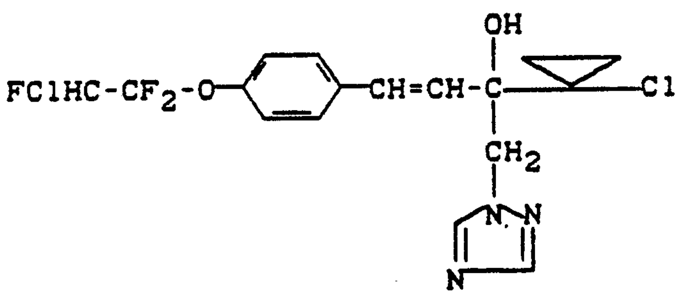

(I-1)

In eine auf 80° C erwärmte Lösung von 2,96 g (0,043 Mol) 1,2,4-Triazol und 0,48 g (0,0043 Mol) Kalim-tert.-butylat in 30 ml Dimethylformamid werden 3,90 g (0,011 Mol) 2-(1-Chlor-cyclopropyl)-2-[4-(2-chlor-1,1,2-trifluorethoxy)-phenylethenyl]-oxiran in 20 ml Dimethylformamid unter Rühren eingetropft. Das Reaktionsgemisch wird 12 Stunden bei 80° C nachgerührt und dann unter vermindertem Druck durch Abziehen des Lösungsmittels eingeengt. Man nimmt den Rückstand in Ethylacetat auf, wäscht die entstehende Lösung dreimal mit Wasser, trocknet über Natriumsulfat und engt unter vermindertem Druck ein. Der verbleibende Rückstand wird mit einem Gemisch aus Cyclohexan: Essigester = 1:1 über eine Kieselgel-Säule chromatographiert. Nach dem Eindampfen des Eluates erhält man 3,2 g (69 % der Theorie) an 2-(1-Chlorcyclopropyl)-4-[4-(2-chlor-1,1,2- trifluorethoxy)-phenyl]-1-(1,2,4-triazol-1-yl)-but-3-en-2-ol in Form einer Festsubstanz mit dem Schmelzpunkt 113-118° C.

Herstellung von Ausgangssubstanzen:

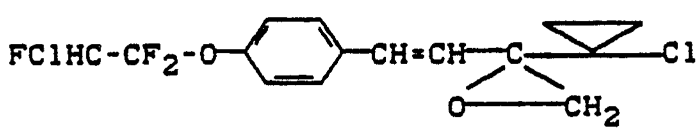

(II-1)

Eine Suspension von 0,75 g (0,031 Mol) Natriumhydrid in 25 ml eines Gemisches aus Dimethylsulfoxid: Tetrahydrofuran = 3:2 wird bei -15° C unter Rühren mit einer Lösung von 6,35 g (0,031 Mol) Trimethylsulfoxonium-iodid in 20 ml Dimethylsulfoxid versetzt. Nach dem Erwärmen des Reaktionsgemisches auf 0° C wird unter Rühren eine Lösung von 9,6 g (0,028 Mol) 1-Chlor-cyclopropyl-4-(2-chlor-1,1,2-trifluorethoxy)-phenyl-ethenyl-keton in 15 ml Dimethylsulfoxid zugetropft. Es wird 15 Minuten bei 0° C und 45 Minuten bei Raumtemperatur nachgerührt. Anschließend wird das Reaktionsgemisch in 100 ml Eiswasser eingegossen und dreimal mit Cyclohexan extrahiert. Die vereinigten organischen Phasen werden einmal mit Wasser gewaschen, über Natriumsulfat getrocknet und dann unter vermindertem Druck eingeengt. Man erhält auf diese Weise 8,89 g (89 % der Theorie) an 2-(1-Chlor-cyclopropyl)-2-[4-(2-chlor-1,1,2-trifluorethoxy)-phenylethenyl]-oxiran in Form eines öligen Produktes, das ohne zusätzliche Reinigung weiter umgesetzt wird.

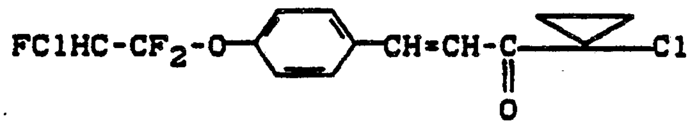

(V-1)

Eine Lösung von 24,3 g (0,102 Mol) 4-(2-Chlor-1,1,2-trifluorethoxy)-benzaldehyd und 12,1.g (0,102 Mol) 1-Chlor-cyclopropyl-methyl-keton in 100 ml Tetrahydrofuran wird bei Raumtemperatur mit 0,6 g (0,011 Mol) Kaliumhydroxid-Pulver versetzt und danach 8 Stunden bei 40 bis 50° C gerührt. Anschließend wird das Reaktionsgemisch in eine gesättigte, wäßrige Ammoniumchlorid-Lösung gegossen und zweimal mit Cyclohexan extrahiert. Die vereinigten organischen Phasen werden über Natriumsulfat getrocknet und unter vermindertem Druck eingeengt. Der verbleibende Rückstand wird mit einem Gemisch aus Cyclohexan: Ethylacetat = 10:1 als Laufmittel an Kieselgel chromatcgraphiert. Durch Eindampfen des Eluates erhält man 17 g (49 % der Theorie) an 1-Chlor-cyclopropyl-[4-(2-chlor-1,1,2-trifluorethoxy)-phenyl-ethenyl]-keton in Form eines öligen Produktes.

IR-Spektrum ($CHCl_3$):
Banden bei 1580, 1600 und 1670 $cm^{-1}$

Beispiel 2

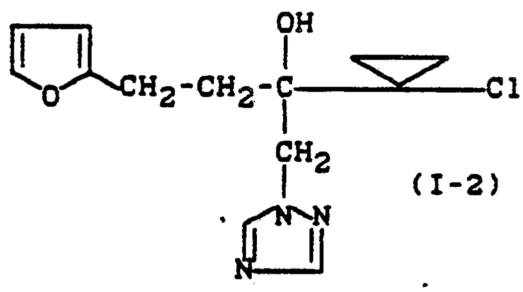

(I-2)

In eine auf 80° C erwärmte Lösung von 10,43 g (0,151 Mol) 1,2,4-Triazol und 1,12 g (0,01 Mol) Kalium-tert.-butylat in 80 ml Dimethylformamid werden 10,71 g (0,054 Mol) 2-(1-Chlor-cyclopropyl)-2-(furan-2-yl-ethyl)-oxiran unter Rühren eingetropft. Das Reaktionsgemisch wird 6 Stunden bei 80° C nachgerührt und dann durch Abziehen des Lösungsmittels unter vermindertem Druck eingeengt. Man nimmt den verbleibenden Rückstand in Ethylacetat auf, wäscht die organische Phase zweimal mit Wasser, trocknet über Natriumsulfat und engt unter vermindertem Druck ein. Der verbleibende Rückstand wird mit einem Gemisch aus Cyclohexan: Essigester 1:1 als Laufmittel an Kieselgel chromatographiert. Nach dem Einengen des Eluates erhält man 7,87 g (55 % der Theorie) an 2-(1-Chlor-cyclopropyl)-4-(furan-2-yl-ethyl)-1-(1,2,4-triazol-1-yl)-butan-2-ol in Form einer Festsubstanz vom Schmelzpunkt 83-86° C.

Herstellung von Ausgangssubstanzen

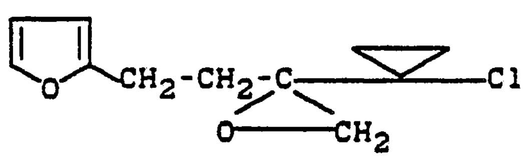

(II-2)

In eine auf -15° C gekühlte Suspension von 1,33 g (0,055 Mol) Natriumhydrid in 75 ml eines Gemisches aus Dimethylsulfoxid: Tetrahydrofuran = 1:2 wird eine Lösung von 11,3 g (0,055 Mol) Trimethylsulfonium-iodid in 50 ml Dimethylsulfoxid unter Rühren eingetropft. Nach dem Erwärmen des Reaktionsgemisches auf 0° C wird unter Rühren eine Lösung von 10,0 g (0,05 Mol) 1-(Chlor-cyclopropyl)-(furan-2-yl-ethyl)-keton in 20 ml Dimethylsulfoxid eingetropft. Es wird 10 Minuten bei 0° C und 1 Stunde bei Raumtemperatur nachgerührt. Anschließend wird das Reaktionsgemisch auf Eiswasser geschüttet und dreimal mit Cyclohexan extrahiert. Die vereinigten organischen Phasen werden über Natriumsulfat getrocknet und unter vermindertem Druck eingeengt. Man erhält auf diese Weise 10,27 g (96 % der Theorie) an 2-(1-Chlor-cyclopropyl)-2-(furan-2-yl-ethyl)-oxiran in Form eines öligen Produktes, das ohne zusätzliche Reinigung weiter umgesetzt wird.

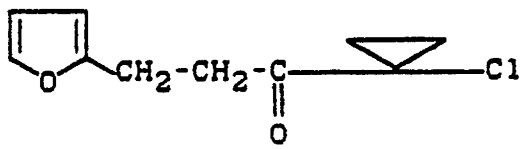 (V-2)

In einen 0,3 l Autoklaven werden 740 mg Tris-triphenylphosphin-rhodiumchlorid gegeben. Nach dem Spülen des Autoklaven mit Stickstoff wird eine luftfreie Lösung von 17 g 1-Chlor-cyclopropyl-4-(furan-2-yl-)-ethenyl-keton in 150 ml Toluol hinzugefügt und unter einem Wasserstoffdruck von 30 bar auf 50 °C erhitzt. Der Wasserstoffdruck wird zwischen 50 und 60 bar gehalten bis die Gasaufnahme beendet ist. Anschließend läßt man 1 Stunde nachreagieren. Zur Aufarbeitung wird das Lösungsmittel unter vermindertem Druck abgezogen. Der verbleibende Rückstand mit Dichlormethan als Laufmittel an Kieselgel chromatographiert. Nach dem Eindampfen des Eluates erhält man 14,7 g (86 % der Theorie) an 1-Chlor-cyclopropyl-4-(furan-2-yl)-ethyl-keton in Form eines öligen Produktes.

$^1$H-NMR-Spektrum (200 MHz, $CDCl_3$):
$\delta$ = 1,35 (m, 2H); 1,65 (m, 2H); 2,9 (t, 2H); 3,2 (t, 2H); 6,0 (m, 1H); 6,3 (m, 1H); 7,3 (m, 1H).

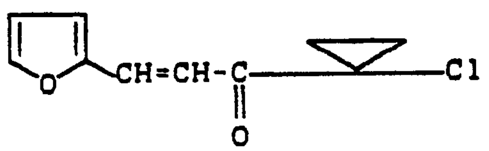 (V-3)

Eine Lösung von 20,0 g (0,169 Mol) 1-Chlor-cyclopropylmethyl-keton und 14,0 ml (0,169 Mol) Furan-2-carbaldehyd in 100 ml Tetrahydrofuran wird bei Raumtemperatur mit 0,95 g (0,017 Mol) Kaliumhydroxidpulver versetzt und 18 Stunden bei Raumtemperatur gerührt. Das Reaktionsgemisch wird in gesättigte, wäßrige Ammoniumchlorid-Lösung gegossen und dreimal mit Cyclohexan extrahiert. Die vereinigten organischen Phasen werden über Natriumsulfat getrocknet und unter vermindertem Druck eingeengt. Man erhält auf diese Weise 34,0 g (100 % der Theorie) an 1-Chlor-cyclopropyl-4-(furan-2-yl)-ethenyl-keton in Form eines Öles.

IR-Spektrum (Film):
Banden bei 1550, 1600 und 1680 $cm^{-1}$

Beispiel 3

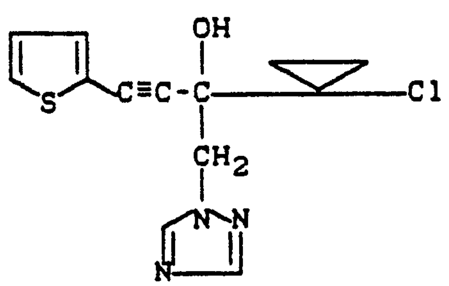 (I-3)

Eine Suspension von 100 g (0,72 Mol) Kaliumcarbonat und 24,8 g (0,36 Mol) 1,2,4-Triazol in 300 ml Aceton wird mit 47,0 g (0,18 Mol) 1-Chlor-2-(1-chlor-cyclopropyl)-4-(thiophen-2-yl)-but-3-in-2-ol versetzt und 2 Stunden unter Rückfluß erhitzt. Anschließend wird das Lösungsmittel unter vermindertem Druck abgezogen, der verbleibende Rückstand in Wasser aufgenommen und dreimal mit Dichlormethan extrahiert. Die

vereinigten organischen Phasen werden über Natriumsulfat getrocknet und unter vermindertem Druck eingeengt. Der verbleibende Rückstand wird mit einem Gemisch aus Cyclohexan/Ethylacetat = 1:1 als Laufmittel an Kieselgel chromatographiert. Nach dem Eindampfen des Eluates erhält man 7,9 g (15 % der Theorie) an 2-(1-Chlor-cyclopropyl)-4-(thiophen-2-yl)-1-(1,2,4-triazol-1-yl)-3-butin-2-ol in Form eines öligen Produktes.

$^1$H-NMR-Spektrum (200 MHz, $CDCl_3$):
δ = 1,00-130 (m, 4H); 4,78 (AB-System, 2H); 6,98 (m, 1H); 7,18 (m, 1H); 7,30 (m, 1H); 7,96 (s, 1H); 8,36 (s, 1H).

Herstellung von Ausgangssubstanzen

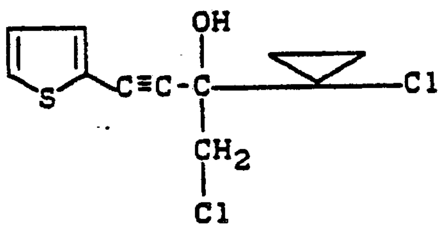
(IV-1)

4,9 g (0,203 Mol) Magnesiumspäne in 100 ml Diethylether werden langsam mit 22,2 g (0,203 Mol) Ethylbromid versetzt. Nach beendeter Zugabe wird das Reaktionsgemisch 30 Minuten unter Rückfluß gekocht, dann auf Raumtemperatur abgekühlt und anschließend unter Rühren tropfenweise mit einer Lösung von 20 g (0,185 Mol) 2-Thienylethin in 50 ml Diethylether versetzt. Es wird 1 Stunde unter Rückfluß erhitzt und erneut auf Raumtemperatur abgekühlt. Man tropft unter Rühren 28,3 g (0,185 Mol) 1-Chlor-cyclopropyl-chlormethyl-keton hinzu und erhitzt dann weitere 2 Stunden unter Rückfluß. Das Reaktionsgemisch wird nach dem Abkühlen auf Raumtemperatur in eine gesättigte, wäßrige Ammoniumchlorid-Lösung gegossen und dreimal mit Diethylether extrahiert. Die vereinigten organischen Phasen werden einmal mit Wasser gewaschen, über Natriumsulfat getrocknet und unter vermindertem Druck eingeengt. Man erhält auf diese Weise 47,0 g (97 % der Theorie) an 1-Chlor-2-(1-chlor-cyclopropyl)-4-(thiophen-2-yl)-but-3-in-2-ol in Form eines Öles, das ohne zusätzliche Reinigung weiter umgesetzt wird.

Beispiel 4

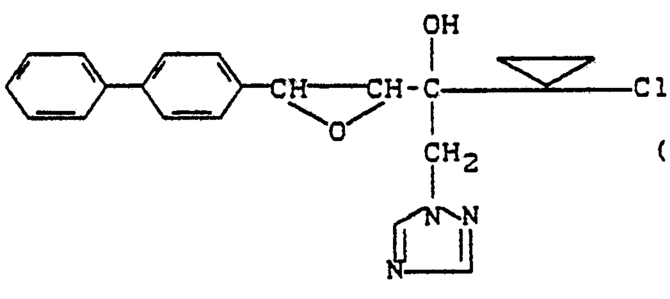
(I-4)

Eine Lösung von 4,0 g (0,011 Mol) 4-Biphenyl-2-(1-chlor-cyclopropyl)-1-(1,2,4-triazol-1-yl)-but-3-en-2-ol in 50 ml Dichlormethan wird bei 0 °C mit 2,26 g (0,013 Mol) 3-Chlor-perbenzoesäure versetzt und 5 Stunden bei Raumtemperatur gerührt. Anschließend wird das Reaktionsgemisch dreimal mit halbgesättigter, wäßriger Natriumcarbonat-Lösung gewaschen, über Natriumsulfat getrocknet und dann unter verminderten Druck eingeengt. Der verbleibende Rückstand wird mit einem Gemisch aus Cyclohexan/Ethylacetat = 1:1 als Laufmittel an Kieselgel chromatographiert. Man erhält auf diese Weise 2,7 g (64 % der Theorie) an 4-Biphenyl-2-(1-chlor-cyclopropyl)-3,4-epoxy-1-(1,2,4-triazol-1-yl)-butan-2-ol in Form eines Feststoffes mit einem Schmelzpunkt von 250-254 °C.

Nach den in den vorhergehenden Beispielen angegebenen Methoden werden auch die in der folgenden

Tabelle 1 aufgeführten Verbindungen der Formel

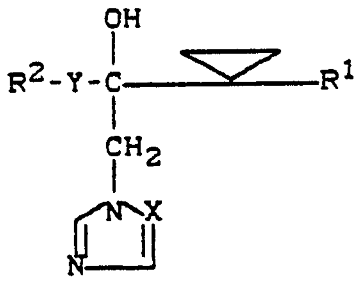

(I)

hergestellt.

**Tabelle 1**

| Beispiel Nr. | Verb.-Nr. | $R^2$ | Y | X | $R^1$ | Physikalische bzw. spektroskopische Konst. |
|---|---|---|---|---|---|---|
| 5 | I-5 | 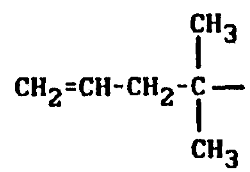 | -CH=CH- | N | Cl | IR: 1700, 1730, 3000-3400 $cm^{-1}$ |
| 6 | I-6 | 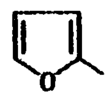 | -CH=CH- | N | Cl | IR: 1730, 3100-3500 $cm^{-1}$ |
| 7 | I-7 | 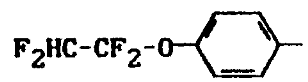 | -CH=CH- | N | Cl | IR: 1730, 3200-3400 $cm^{-1}$ |
| 8 | I-8 | 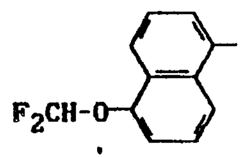 | -CH=CH- | N | Cl | IR: 1600, 1630, 3200-3400 $cm^{-1}$ |

<u>Tabelle 1</u> - Fortsetzung -

| Beispiel Nr. | Verb.-Nr. | $R^2$ | Y | X | $R^1$ | Physikalische bzw. spektroskopische Konst. |
|---|---|---|---|---|---|---|
| 9 | I-9 | 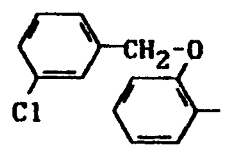 | -CH=CH- | N | Cl | IR: 1600, 1720, 3200-3400 $cm^{-1}$ |
| 10 | I-10 | 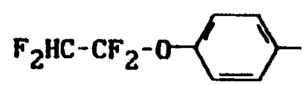 | $-CH_2-CH_2-$ | N | Cl | IR: 1730, 3200-3400 $cm^{-1}$ |
| 11 | I-11 | 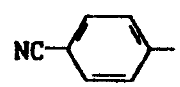 | -CH=CH- | N | Cl | Fp. 51-53° C |
| 12 | I-12 | 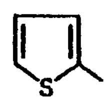 | -CH=CH- | N | Cl | IR: 1730, 3200-3400 $cm^{-1}$ |
| 13 | I-13 | 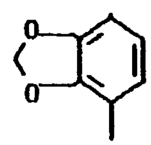 | -CH=CH- | N | Cl | IR: 1670, 1720, 3200-3400 $cm^{-1}$ |

<u>Tabelle 1</u> - Fortsetzung -

| Beispiel Nr. | Verb.-Nr. | $R^2$ | Y | X | $R^1$ | Physikalische bzw. spektroskopische Konst. |
|---|---|---|---|---|---|---|
| 14 | I-14 | 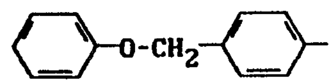 | -CH=CH- cis | N | Cl | IR: 1600, 1660, 3200-3400 $cm^{-1}$ |
| 15 | I-15 | 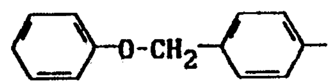 | -CH=CH- trans | N | Cl | IR: 1600, 1720, 3200-3400 $cm^{-1}$ |
| 16 | I-16 | 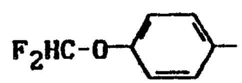 | -CH=CH- | N | Cl | Fp. 48-49° C |
| 17 | I-17 | 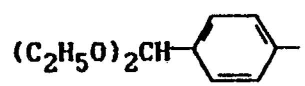 | -CH=CH- | N | Cl | IR: 1720 $cm^{-1}$ 3200-3400 $cm^{-1}$ |
| 18 | I-18 | 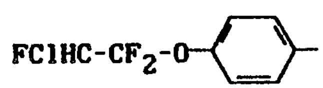 | -CH=CH- | N | 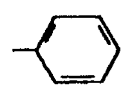 | IR: 1680, 1720, 3200-3400 $cm^{-1}$ |

<u>Tabelle 1</u> - Fortsetzung -

| Beispiel Nr. | Verb.- Nr. | $R^2$ | Y | X | $R^1$ | Physikalische bzw. spektroskopische Konst. |
|---|---|---|---|---|---|---|
| 19 | I-19 | 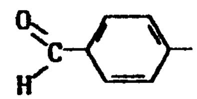 | -CH=CH- | N | Cl | Fp: 87-88°C |
| 20 | I-20 | 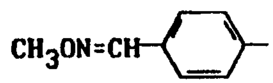 | -CH=CH- | N | Cl | IR: 1700, 3200-3400 $cm^{-1}$ |
| 21 | I-21 | 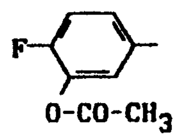 | -CH=CH- | N | Cl | Fp: 143-145°C |
| 22 | I-22 | 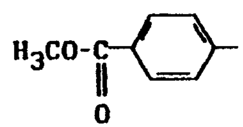 | -CH=CH- | N | Cl | Fp: 128°C |

<u>Tabelle 1</u> - Fortsetzung -

| Beispiel Nr. | Verb.-Nr. | $R^2$ | Y | X | $R^1$ | Physikalische bzw. spektroskopische Konst. |
|---|---|---|---|---|---|---|
| 23 | I-23 | 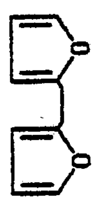 | $-CH_2-CH_2$ | N | Cl | IR: 1730, 3200-3400 $cm^{-1}$ |
| 24 | I-24 | 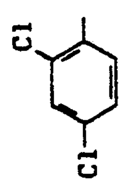 | 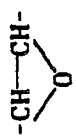 | N | Cl | IR: 1600, 1720 3200-3500 $cm^{-1}$ |

In den folgenden Verwendungsbeispielen wurden die Verbindungen der nachstehend angegebenen Formeln als Vergleichssubstanzen eingesetzt:

(A) =

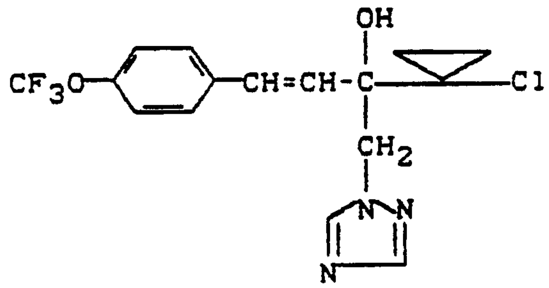

(B) =

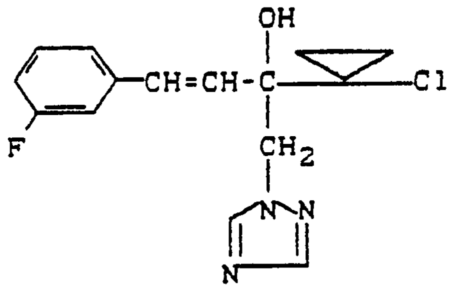

(C) =

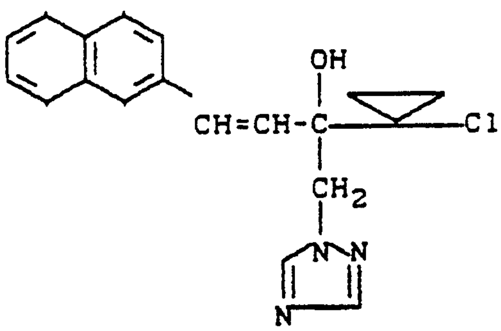

(D) =

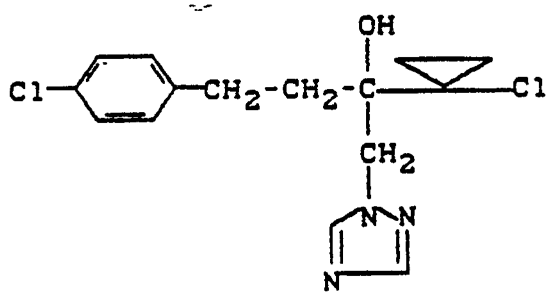

(E) =

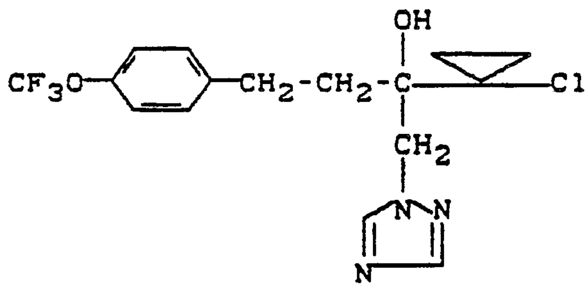

(F) =

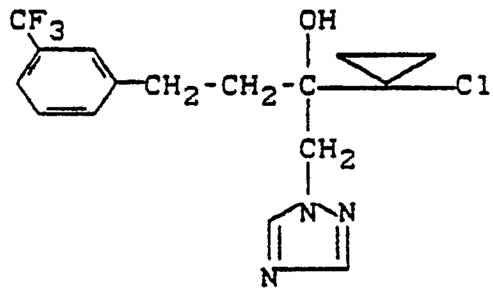

(G) =

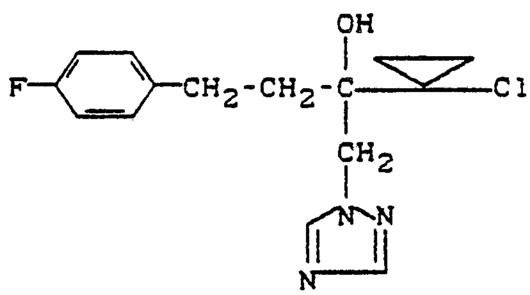

(H) =

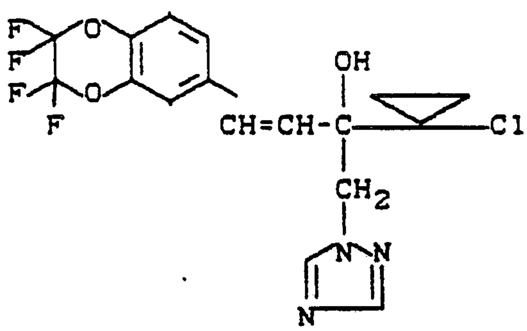

(J) =

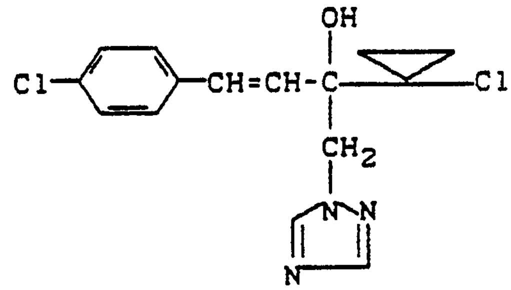

(K) =

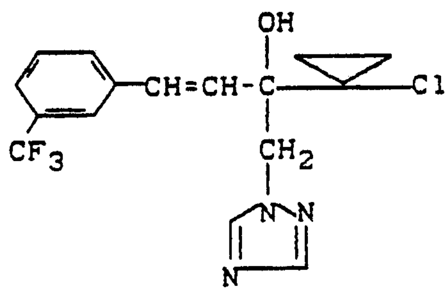

Die Vergleichssubstanzen sind aus der EP-OS 0 298 332 bekannt.

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-7) eine wesentlich bessere Wirksamkeit als die Vergleichssubstanzen (A), (B) und (C).

Beispiel B

Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigt die erfindungsgemäße Verbindung (I-10) eine wesentlich bessere Wirksamkeit als die Vergleichssubstanzen (D), (E) und (F).

Beispiel C

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

In diesem Test zeigt der erfindungsgemäße Stoff (I-10) eine wesentlich bessere Wirksamkeit als die Vergleichssubstanzen (D), (F) und (G).

Außerdem zeigen in diesem Teset die erfindungsgemäßen Verbindungen (I-1), (I-7), (I-8), (I-14), (I-19), (I-20) und (I-22) eine wesentlich bessere Wirksamkeit als die Vergleichssubstanz (H).

Beispiel D

Leptosphaeria nodorum-Test (Weizen)/kurativ

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Sporensuspension von Leptosphaeria nodorum besprüht. Die Pflanzen verbleiben 48 Stunden bei 20 °C und 100 % rel. Luftfeuchtigkeit in einer Inkubationskabine. Anschließend besprüht man die Pflanzen mit der Wirkstoffkonzentration taufeucht.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

In diesem Test zeigt die erfindungsgemäße Verbindung (I-7) eine wesentlich bessere Wirksamkeit als die Vergleichssubstanz (J).

Beispiel E

Pyrenophora teres-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigen die erfindungsgemäßen Verbindungen (I-1) und (I-13) eine wesentlich bessere Wirksamkeit als die Vergleichssubstanzen (B) und (K).

## Beispiel F

Fusarium nivale-Test (Roggen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10 °C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

In diesem Test zeigen die erfindungsgemäßen Verbindungen (I-1) und (I-7) eine wesentlich bessere Wirksamkeit als die Vergleichssubstanz (K).

## Beispiel G

Venturia-Test (Apfel) / kurativ

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspensions des Apfelschorferregers (Venturia inaequalis) inokuliert. Die Pflanzen verbleiben 1 Tag bei 20 °C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigt die erfindungsgemäße Verbindung (I-10) eine wesentlich bessere Wirkung als die Vergleichssubstanzen (F) und (G).

Außerdem zeigen in diesem Test die erfindungsgemäßen Verbindungen (I-1), (I-7) und (I-8) eine wesentlich bessere Wirkung als die Vergleichssubstanzen (B) und (K).

## Ansprüche

1. Hydroxyethyl-cyclopropyl-azolyl-Derivate der Formel

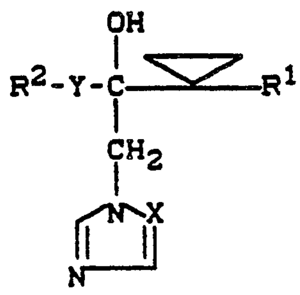 (I)

in welcher
$R^1$ für Halogen, Phenyl oder die Gruppierung $-Z-R^3$ steht, worin
Z für Sauerstoff, Schwefel, SO oder $SO_2$ steht und
$R^3$ für Alkyl, Phenyl oder Benzyl steht,
$R^2$ für Alkenyl, gegebenenfalls substituiertes Furyl, gegebenenfalls substituiertes Thienyl oder den Rest der Formel

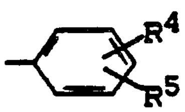

steht, worin
$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und
$R^5$ für Wasserstoff oder Halogen steht, oder
$R^4$ und $R^5$ in ortho-Position verknüpft sind und gemeinsam für $-O-CH_2-O-$ stehen,
oder
$R^2$ für den Rest der Formel

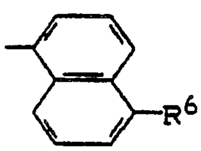

steht,
worin
$R^6$ für Difluormethoxy, 1,1,2,2-Tetrafluorethoxy oder 1,1,2 -Trifluor-2-chlorethoxy steht,
oder
$R^2$ auch für gegebenenfalls durch Halogen und/oder Phenyl substituiertes Phenyl steht, wenn Y für die Gruppierung

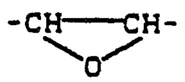

steht,
X für Stickstoff oder eine CH-Gruppe steht und
Y für die Gruppierungen

$-CH_2-CH_2-$,

$-CH=CH-$, $-C\equiv C-$ oder

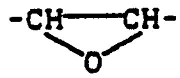

steht,

sowie deren Säureadditionssalze und Metallsalzkomplexe.

2. Verfahren zur Herstellung von Hydroxyethylcyclopropyl-azolyl-Derivaten der Formel

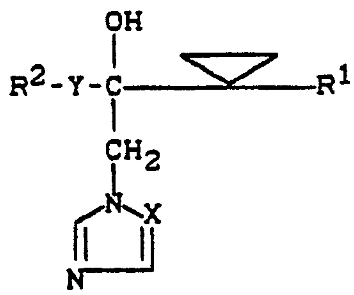

(I)

in welcher

$R^1$ für Halogen, Phenyl oder die Gruppierung $-Z-R^3$ steht, worin

Z für Sauerstoff, Schwefel, SO oder $SO_2$ steht und

$R^3$ für Alkyl, Phenyl oder Benzyl steht,

$R^2$ für Alkenyl, gegebenenfalls substituiertes Furyl, gegebenenfalls substituiertes Thienyl oder den Rest der Formel

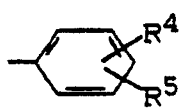

steht, worin

$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und

$R^5$ für Wasserstoff oder Halogen steht, oder

$R^4$ und $R^5$ in ortho-Position verknüpft sind und gemeinsam für $-O-CH_2-O-$ stehen,

oder

$R^2$ für den Rest der Formel

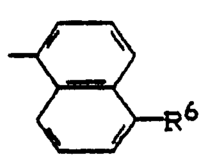

steht,

worin

$R^6$ für Difluormethoxy, 1,1,2,2-Tetrafluorethoxy oder 1,1,2 -Trifluor-2-chlorethoxy steht,

oder

$R^2$ auch für gegebenenfalls durch Halogen und/oder Phenyl substituiertes Phenyl steht, wenn Y für die Gruppierung

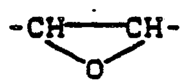

steht,
X für Stickstoff oder eine CH-Gruppe steht und
Y für die Gruppierungen

$-CH_2-CH_2-$,
$-CH=CH-$, $-C\equiv C-$ oder
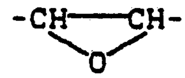

steht,
sowie von deren Säureadditions-Salzen und Metallsalz-Komplexen, dadurch gekennzeichnet, daß man
a) Oxirane der Formel

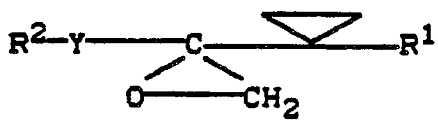
(II)

in welcher
$R^1$, $R^2$ und Y die oben angegebene Bedeutung haben,
mit Azolen der Formel

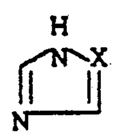
(III)

in welcher
X die oben angegebene Bedeutung hat,
in Gegenwart eines Säurebindemittels und in Gegenwart eines Verdünnungsmittels umsetzt,
oder
b) Ethanol-Derivate der Formel

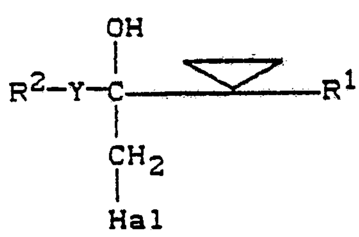
(IV)

in welcher
$R^1$, $R^2$ und Y die oben angegebene Bedeutung haben, und
Hal für Chlor, Brom oder Iod steht,
mit Azolen der Formel

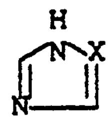

(III)

in welcher
X die oben angegebene Bedeutung hat,
in Gegenwart eines Säurebindemittels und in Gegenwart ein Verdünnungsmittels umsetzt,
oder
c) Hydroxyethyl-cyclopropyl-azolyl-Derivate der Formel

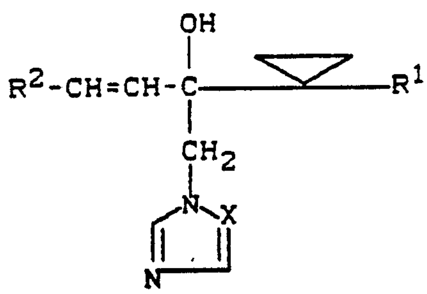

(Ia)

in welcher
$R^1$, $R^2$ und X die oben angegebene Bedeutung haben,
mit einem zur Expoxidierung geeigneten Reagenz gegebenenfalls in Gegenwart eines Verdünnungsmittels umsetzt,
und gegebenenfalls anschließend an die so erhaltenen Verbindungen der Formel (I) eine Säure oder ein Metallsalz addiert.

3. Mikrobizide Mittel, gekennzeichnet durch einen Gehalt an mindestens einem Hydroxyethyl-cyclopropyl-azolyl-Derivat der Formel (I) gemäß Anspruch 1 bzw. an einem Säureadditions-Salz oder Metallsalz-Komplex eines Hydroxyethyl-cyclopropylazolyl-Derivates der Formel (I).

4. Verwendung von Hydroxyethyl-cyclopropyl-azolyl-Derivaten der Formel (I) gemäß Anspruch 1 bzw. von deren Säureadditions-Salzen und Metallsalz-Komplexen als Mikrobizide im Pflanzenschutz und im Materialschutz.

5. Oxirane der Formel

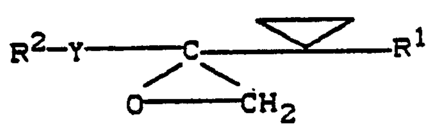

(II)

in welcher
$R^1$ für Halogen, Phenyl oder die Gruppierung $-Z-R^3$ steht, worin
Z für Sauerstoff, Schwefel, SO oder $SO_2$ steht und
$R^3$ für Alkyl, Phenyl oder Benzyl steht,
$R^2$ für Alkenyl, gegebenenfalls substituiertes Furyl, gegebenenfalls substituiertes Thienyl oder den Rest der Formel

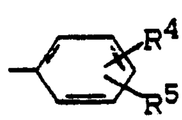

steht, worin
$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder

für gegebenenfalls im Phenylteil durch Halogen substituiertes Benzyloxy steht und
$R^5$ für Wasserstoff oder Halogen steht, oder
$R^4$ und $R^5$ in ortho-Position verknüpft sind und gemeinsam für $-O-CH_2-O-$ stehen,
oder
$R^2$ für den Rest der Formel

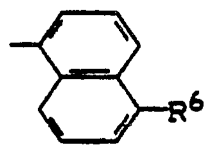

steht,
worin
$R^6$ für Difluormethoxy, 1,1,2,2-Tetrafluorethoxy oder 1,1,2 -Trifluor-2-chlorethoxy steht,
oder
$R^2$ auch für gegebenenfalls durch Halogen und/oder Phenyl substituiertes Phenyl steht, wenn Y für die Gruppierung

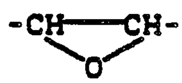

steht,
und
Y für die Gruppierungen

$-CH_2-CH_2-$,
$-CH=CH-$, $-C \equiv C-$ oder

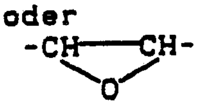

steht.
6. Verfahren zur Herstellung von Oxiranen der Formel

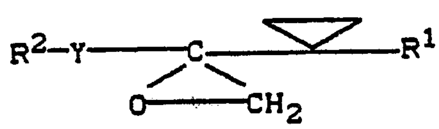

(II)

in welcher
$R^1$ für Halogen, Phenyl oder die Gruppierung $-Z-R^3$ steht, worin
Z für Sauerstoff, Schwefel, SO oder $SO_2$ steht und
$R^3$ für Alkyl, Phenyl oder Benzyl steht,
$R^2$ für Alkenyl, gegebenenfalls substituiertes Furyl, gegebenenfalls substituiertes Thienyl oder den Rest der Formel

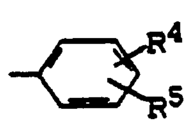

steht, worin

$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und
$R^5$ für Wasserstoff oder Halogen steht, oder
$R^4$ und $R^5$ in ortho-Position verknüpft sind und gemeinsam für $-O-CH_2-O-$ stehen,
oder
$R^2$ für den Rest der Formel

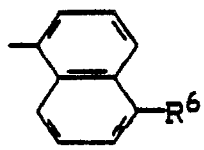

steht,
worin
$R^6$ für Difluormethoxy, 1,1,2,2-Tetrafluorethoxy oder 1,1,2,-Trifluor-2-chlorethoxy steht,
oder
$R^2$ auch für gegebenenfalls durch Halogen und/oder Phenyl substituiertes Phenyl steht, wenn Y für die Gruppierung

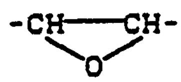

steht,
und
Y für die Gruppierungen

$-CH_2-CH_2-$,

$-CH=CH-$, $-C\equiv C-$ oder

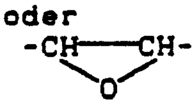

steht,
dadurch gekennzeichnet, daß man
d) Cyclopropylketone der Formel

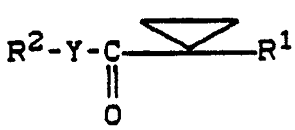

(V)

in welcher
$R^1$, $R^2$ und Y die oben angegebene Bedeutung haben,
entweder
α) mit Dimethyloxosulfonium-methylid der Formel

$$\overset{\delta^{\oplus}\,\delta^{\ominus}}{(CH_3)_2SOCH_2} \qquad (VI)$$

oder

β) mit Dimethylsulfonium-methylid der Formel

$$\overset{\delta\oplus\ \delta\ominus}{(CH_3)_2S\ \ CH_2} \qquad (VII)$$

in Gegenwart eines Verdünnungsmittels umsetzt, oder indem man

e) Carbinole der Formel

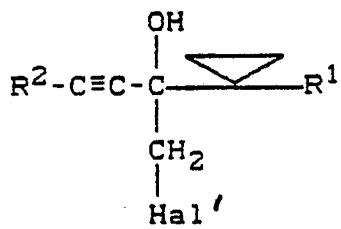

(IVa)

in welcher

$R^1$ und $R^2$ die oben angegebene Bedeutung haben und

Hal′ für Chlor oder Brom steht,

mit Basen in Gegenwart eines Verdünnungsmittels umsetzt.

7. Cyclopropylketone der Formel

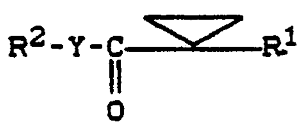

(V)

in welcher

$R^1$ für Halogen, Phenyl oder die Gruppierung $-Z-R^3$ steht, worin

Z für Sauerstoff, Schwefel, SO oder $SO_2$ steht und

$R^3$ für Alkyl, Phenyl oder Benzyl steht,

$R^2$ für Alkenyl, gegebenenfalls substituiertes Furyl, gegebenenfalls substituiertes Thienyl oder den Rest der Formel

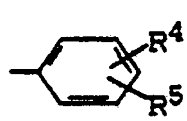

steht, worin

$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und

$R^5$ für Wasserstoff oder Halogen steht, oder

$R^4$ und $R^5$ in ortho-Position verknüpft sind und gemeinsam für $-O-CH_2-O-$ stehen,

oder

$R^2$ für den Rest der Formel

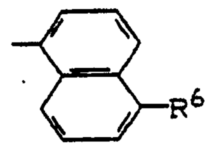

steht,
worin
$R^6$ für Difluormethoxy, 1,1,2,2-Tetrafluorethoxy oder 1,1,2 -Trifluor-2-chlorethoxy steht,
oder
$R^2$ auch für gegebenenfalls durch Halogen und/oder Phenyl substituiertes Phenyl steht, wenn Y für die Gruppierung

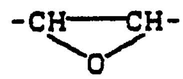

steht,
Y für die Gruppierungen

$-CH_2-CH_2-$,

$-CH=CH-$, $-C\equiv C-$ oder

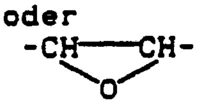

steht.

8. Verfahren zur Herstellung von Cyclopropylketonen der Formel

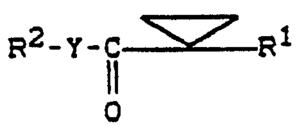

(V)

in welcher
$R^1$ für Halogen, Phenyl oder die Gruppierung $-Z-R^3$ steht, worin
Z für Sauerstoff, Schwefel, SO oder $SO_2$ steht und
$R^3$ für Alkyl, Phenyl oder Benzyl steht,
$R^2$ für Alkenyl, gegebenenfalls substituiertes Furyl, gegebenenfalls substituiertes Thienyl oder den Rest der Formel

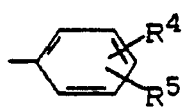

steht, worin
$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und
$R^5$ für Wasserstoff oder Halogen steht, oder
$R^4$ und $R^5$ in ortho-Position verknüpft sind und gemeinsam für $-O-CH_2-O-$ stehen,
oder
$R^2$ für den Rest der Formel

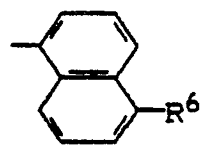

steht,
worin
$R^6$ für Difluormethoxy, 1,1,2,2-Tetrafluorethoxy oder 1,1,2,-Trifluor-2-chlorethoxy steht,
oder
$R^2$ auch für gegebenenfalls durch Halogen und/oder Phenyl substituiertes Phenyl steht, wenn Y für die Gruppierung

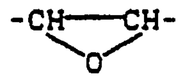

steht,
Y für die Gruppierungen

$-CH_2-CH_2-$,
$-CH=CH-$, $-C\equiv C-$ oder

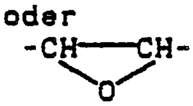

steht,
dadurch gekennzeichnet, daß man

f) Aldehyde der Formel
$R^2$-CHO (VIII)
in welcher
$R^2$ die oben angegebene Bedeutung hat,
mit Methylencyclopropyl-ketonen der Formel

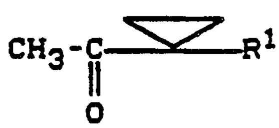

(IX)

in welcher
$R^1$ die oben angegebene Bedeutung hat,
in Gegenwart eines Katalysators und in Gegenwart eines Verdünnungsmittels umsetzt und gegebenenfalls die dabei entstehenden Cyclopropylketone der Formel

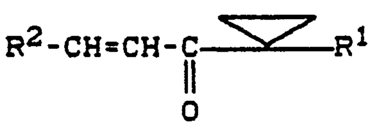

(V-a)

in welcher
$R^1$ und $R^2$ die oben angegebene Bedeutungen haben,
entweder

α) in Gegenwart eines Katalysators und in Gegenwart eines Verdünnungsmittels hydriert,
oder
β) mit einem zur Epoxidierung geeigneten Reagenz gegebenenfalls in Gegenwart eines Verdünnungsmittels umsetzt,
oder indem man
g) Acetylene der Formel
$R^2$-C≡CH (X)
in welcher
$R^2$ die oben angegebene Bedeutung hat,
mit Säurehalogeniden der Formel

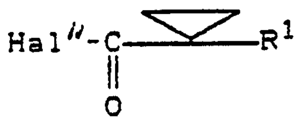

(XI)

in welcher
$R^1$ die oben angegebene Bedeutung hat und
Hal'' für Chlor oder Brom steht,
in Gegenwart eines Katalysators und in Gegenwart eines Verdünnungsmittels umsetzt.

9. Ethanol-Derivate der Formel

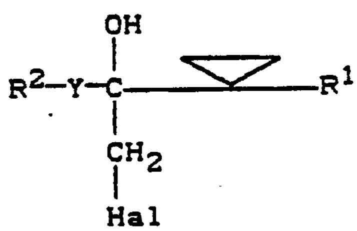

(IV)

in welcher
$R^1$ für Halogen, Phenyl oder die Gruppierung -Z-$R^3$ steht, worin
Z für Sauerstoff, Schwefel, SO oder $SO_2$ steht und
$R^3$ für Alkyl, Phenyl oder Benzyl steht,
$R^2$ für Alkenyl, gegebenenfalls substituiertes Furyl, gegebenenfalls substituiertes Thienyl oder den Rest der Formel

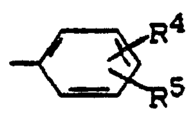

steht, worin
$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und
$R^5$ für Wasserstoff oder Halogen steht, oder
$R^4$ und $R^5$ in ortho-Position verknüpft sind und gemeinsam für -O-$CH_2$-O- stehen,
oder
$R^2$ für den Rest der Formel

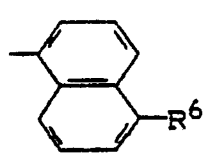

steht,
worin
$R^6$ für Difluormethoxy, 1,1,2,2-Tetrafluorethoxy oder 1,1,2 -Trifluor-2-chlorethoxy steht,

oder
$R^2$ auch für gegebenenfalls durch Halogen und/oder Phenyl substituiertes Phenyl steht, wenn Y für die Gruppierung

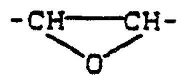

steht,
Y für die Gruppierungen

$-CH=CH-$, $-C\equiv C-$ oder $-CH_2-CH_2-$,

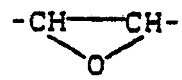

steht
und
Hal für Chlor, Brom oder Iod steht.
10. Verfahren zur Herstellung von Ethanol-Derivaten der Formel

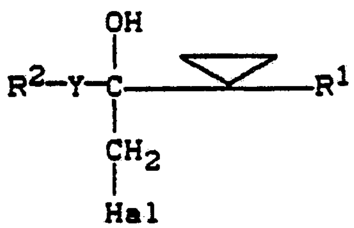

(IV)

in welcher
$R^1$ für Halogen, Phenyl oder die Gruppierung $-Z-R^3$ steht, worin
Z für Sauerstoff, Schwefel, SO oder $SO_2$ steht und
$R^3$ für Alkyl, Phenyl oder Benzyl steht,
$R^2$ für Alkenyl, gegebenenfalls substituiertes Furyl, gegebenenfalls substituiertes Thienyl oder den Rest der Formel

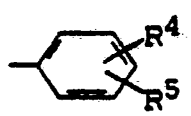

steht worin
$R^4$ für Difluormethoxy, 1,1,2,2-Tetrafluorethoxy, 1,1,2-Trifluor-2-chlor-ethoxy, Cyano, Formyl, Alkoximinoalkyl, Carbalkoxy, Dialkoxyalkyl, gegebenenfalls im Phenylteil durch Halogen substituiertes Phenoxyalkyl oder für gegebenenfalls im Phenylteil durch Halogen substituiertes Benzyloxy steht und
$R^5$ für Wasserstoff oder Halogen steht, oder
$R^4$ und $R^5$ in ortho-Position verknüpft sind und gemeinsam für $-O-CH_2-O-$ stehen,
oder
$R^2$ für den Rest der Formel

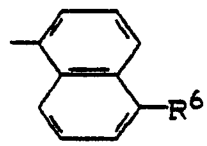

steht,
worin
$R^6$ für Difluormethoxy, 1,1,2,2-Tetrafluorethoxy oder 1,1,2 -Trifluor-2-chlorethoxy steht,
oder
$R^2$ auch für gegebenenfalls durch Halogen und/oder Phenyl substituiertes Phenyl steht, wenn Y für die Gruppierung

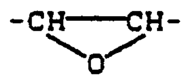

steht,
Y für die Gruppierungen

$-CH_2-CH_2-$, $-CH=CH-$, $-C{\equiv}C-$ oder

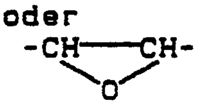

steht,
Hal für Chlor, Brom oder Iod steht,
dadurch gekennzeichnet, daß man
h) Halogenketone der Formel

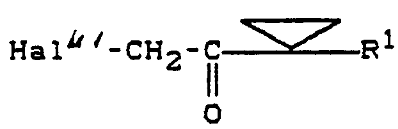

(XII)

in welcher
$R^1$ die oben angegebene Bedeutung hat und
Hal‴ für Chlor oder Brom steht,
mit Acetylensalzen der Formel
$R^2-C{\equiv}C^{\ominus}\ Me^{\oplus}$ (XIII)
in welcher
$R^2$ die oben angegebene Bedeutung hat und
Me für ein Äquivalent eines Metallkations steht,
gegebenenfalls in Gegenwart eines Säurebindemittels und in Gegenwart eines Verdünnungs mittels umsetzt und gegebenenfalls die dabei entstehenden Verbindungen der Formel

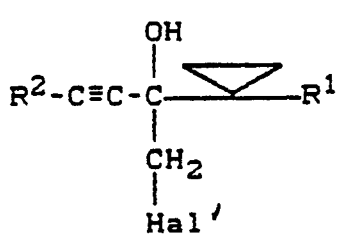

(IVa)

in welcher
$R^1$ und $R^2$ die oben angegebene Bedeutung haben und
Hal' für Chlor oder Brom steht,
ganz oder teilweise hydriert, oder diejenigen Stoffe, in denen Y für -CH=CH- steht, mit Epoxidierungsmitteln gegebenenfalls in Gegenwart eines Verdünnungsmitels umsetzt.

Europäisches Patentamt

# EUROPÄISCHER RECHERCHENBERICHT

Nummer der Anmeldung

EP 90111391.0

| EINSCHLÄGIGE DOKUMENTE | | | KLASSIFIKATION DER ANMELDUNG (Int Cl 5) |
|---|---|---|---|
| Kategorie | Kennzeichnung des Dokuments mit Angabe soweit erforderlich der maßgeblichen Teile | Betrifft Anspruch | |
| P,X | EP - A2 - 0 336 186 (BAYER) * Formel I; Seite 10, Zeile 1; Seite 19, Formel a; Seite 20, Formel b * -- | 1,5,7, 9 | C 07 D 249/08<br>C 07 D 233/60<br>C 07 D 405/06<br>C 07 D 405/14<br>C 07 D 409/06<br>C 07 D 409/14<br>C 07 D 407/06<br>C 07 D 303/08<br>C 07 D 307/12<br>C 07 D 333/16<br>C 07 C 49/163<br>C 07 C 49/237<br>C 07 C 49/225<br>C 07 C 149/32<br>C 07 C 33/50<br>A 01 N 43/56<br>A 01 N 43/653 |
| D,A | EP - A1 - 0 298 332 (BAYER) * Ansprüche 1,6,10 * -- | 1,5,7, 9 | |
| P,A | DE - A1 - 3 819 053 (BASF) * Anspruch 1 * -- | 1,3 | |
| A | DE - A1 - 3 732 385 (BAYER) * Anspruch 1 * -- | 1 | |
| A | EP - A2 - 0 052 424 (JCJ) * Ansprüche 1,11 * -- | 1,3 | RECHERCHIERTE SACHGEBIETE (Int Cl 5) |
| A | CHEMICAL ABSTRACTS, Band 110, Nr. 15, 10. April 1989 Columbus, Ohio, USA BAYDAR AHMET E. et al. "The pyrethrins and related compounds. Part XXXIII. Effective combinations in the gemdimethyl-central group region of non-ester pyrethroids." Seite 265, Spalte 2, Zusammenfassung-Nr. 130 440u & Pestic. Sci. 1988, 23(3), 231-46 -- | 7 | C 07 D 249/00<br>C 07 D 233/00<br>C 07 D 405/00<br>C 07 D 407/00<br>C 07 D 409/00<br>C 07 D 303/00<br>C 07 D 307/00<br>C 07 D 333/00<br>C 07 C 49/00<br>C 07 C 149/00<br>C 07 C 33/00 |
| A | CHEMICAL ABSTRACTS, Band 110, Nr. 7, 13. Februar 1989 Columbus, Ohio, USA | 7 | |

Der vorliegende Recherchenbericht wurde für alle Patentansprüche erstellt.

| Recherchenort | Abschlußdatum der Recherche | Prüfer |
|---|---|---|
| WIEN | 20-09-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-
EP 90111391.0

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int Cl 5) |
|---|---|---|---|
| | CLASS, THOMAS J. et al. "Photochemistry of ethofenprox and three related pyrethroids with ether, alkane and alkene central linkages." Seite 326, Spalte 2, Zusammenfassung-Nr. 53 769d & J. Agric. Food Chem. 1989, 37(1), 216-22 -- | | - |
| A | CHEMICAL ABSTRACTS, Band 99, Nr. 19, 7. November 1983 Columbus, Ohio, USA HEINDEL,NED D. et al. "Transfer hydrogenation of furocoumarin derivatives" Seite 588, Spalte 2, Zusammenfassung-Nr. 158 113t & J. Org. Chem. 1983, 48(21), 3817-19 -- | 7 | |
| A | CHEMICAL ABSTRACTS, Band 77, Nr. 19, 6. November 1972 Columbus, Ohio, USA SHONO,T. et al. "Small ring compounds. XXVII. Migratory aptitude of the cyclopropyl group in the beta-cyclopropylethyl cation systems." Seite 324, Spalte 1, Zusammenfassung-Nr. 125 650h & Tetrahedron Lett. 1972, (31), 3249-52 ---- | 9 | RECHERCHIERTE SACHGEBIETE (Int Cl 5) |

Der vorliegende Recherchenbericht wurde für alle Patentansprüche erstellt

| Recherchenort | Abschlußdatum der Recherche | Prüfer |
|---|---|---|
| WIEN | 20-09-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82